# NEUE EUROPÄISCHE PATENTSCHRIFT

(11) **EP 1 289 642 B2**
(45) Veröffentlichungstag und Bekanntmachung des Hinweises auf die Entscheidung über den Einspruch: **10.10.2012**
(45) Hinweis auf die Patenterteilung: 08.02.2006
(21) Anmeldenummer: 01923626.4
(22) Anmeldetag: 02.03.2001
(51) Int. Cl.: B01J 13/02, B01J 13/22

(54) **NANOKAPSELN MIT EINER POLYELEKTROLYTHÜLLE**
NANOCAPSULES HAVING A POLYELECTROLYTE ENVELOPE
NANOCAPSULES A ENVELOPPE DE POLYELECTROLYTE

(30) Priorität: 02.03.2000 DE 10010264
(43) Veröffentlichungstag der Anmeldung: 12.03.2003
(73) Patentinhaber: Capsulution Pharma AG, 12489 Berlin (DE)
(72) Erfinder: PANZNER, Steffen, 06108 Halle (DE); ENDERT, Gerold, 06114 Halle (DE); ESSLER, Frank, 06108 Halle (DE); BEHRENS, Anja, 50935 Köln (DE); LUTZ, Silke, 06144 Halle (DE); PANZNER, Cornelia, 06108 Halle (DE)
(74) Vertreter: Vossius & Partner
(86) Internationale Anmeldenummer: PCT/EP2001/002397
(87) Internationale Veröffentlichungsnummer: WO 2001/064330

(56) Entgegenhaltungen:
- WO-A-99/47252
- SUKHORUKOV,DONATH,DAVIES,LICHTENFELD,CARUSO ,POPOV,MÖHWALD: 'Stepwise Polyelectrolyte Assembly on Particle Surfaces' POLYMER FOR ADVANCED TECHNOLOGIES Nr. 9, 1998, Seiten 759 - 767, XP784433
- Auszug aus F. Caruso and G. Sukhurokov Coated Colloids:Preparation, Characterization, Assembly and Utilization in: Multilayer Thin Films Sequential Assembly of Nanocomposite Materials. Ed. G. Decher and J.B. Schlenoff Wiley-VCH Verlag, Weinheim, 2003, p331, 334-337

## Beschreibung

Die Erfindung betrifft Nanokapseln, die mit einer in sich stabilen Hüllschicht aus Polyelektrolyten umgeben sind, Verfahren zur Herstellung solcher Strukturen und Verwendungen der Strukturen. Die Erfindung betrifft auch eine Vorrichtung zur Herstellung der Nanokapseln.

Bekannt sind Liposomen als eine biologisch sehr verträgliche Verpackungsform von verschiedenen Wirkstoffen. Die Bestandteile von Liposomen sind in hohen Dosen verträglich und lösen keine oder nur geringe Abwehrreaktionen des Immunsystems aus. Der Verwendung der Liposomen steht jedoch häufig ihre Empfindlichkeit gegenüber mechanischen, thermischen oder biologischen Einwirkungen entgegen.

Eine Verlängerung der Lebensdauer von Liposomen kann durch die Zusammensetzung der liposomalen Membran erreicht werden, allerdings gehen dabei andere wünschenswerte Eigenschaften, wie etwa die Fusionskompetenz, verloren.

Bisher wurden daher vielfältige Anstrengungen unternommen, die schonende und biokompatible Art der Verpackung durch Liposomen mit Hilfe von stabilisierenden Zusätzen für Anwendungen in der Pharmazie und Technik nutzbar zu machen. Ein bekanntes Verfahren zur Erhöhung der Stabilität von Liposomen ist die Dotierung ihrer Oberfläche mit verschiedenen Polymeren, insbesondere mit Polyethylenglykol (PEG). Diese Komponenten bewirken eine sterische Abschirmung der Oberfläche und verhindern so den direkten Angriff lytischer Komponenten, beispielsweise aus dem Blutsystem, an der Membran; z.B. "stealth liposomes" als liposomale Präparationen, bei denen die Liposomen mit einer Hülle aus PEG umgeben sind (D.D.Lasic, Liposomes - from physics to applications").

Andere bekannte Verfahren verwenden einen Schutz der liposomalen Membran durch Aufbringen von Zuckeroligomeren auf die Membranschicht, auch hier wird eine sterische Abschirmung der Membranoberfläche durch die aufgebrachten Komponenten erreicht. Die erhaltenen Strukturen können, im Gegensatz zu den nicht modifizierten Liposomen, eingefroren oder lyophilisiert werden.

In der DE 198 52 928.7 und der WO 00/28972 werden vielseitig modifizierbare, in sich stabile Hüllstrukturen auf liposomalen Templaten offenbart, die sich durch schichtweise Chemisorption von Polymeren oder Biomolekülen herstellen lassen. Das Verfahren erlaubt neben der Herstellung von Hüllschichten und Nanokapseln auch die biokompatible Modifikation und Funktionalisierung der Oberfläche.

Strukturen mit ähnlichem Aufbau können alternativ auch durch schichtweise Polyelektrolyt- Selbstassemblierung auf kolloidalen Templaten erzeugt werden (Caruso, F. (1998) Science 282:1111-1113, DE 198 12 083 A1, EP 0972563 A1 bzw. WO 99/47253).

Aus der WO 99/47252 A ist ein Verfahren zur Herstellung von Nano- oder Mikrokapseln bekannt, bei denen die Hülle mehrere Elektrolytschichten umfasst. Bei dem Verfahren gemäß WO 99/47252 wird überschüssiges Beachichtungspolymer mit einer Filtermembran abgetrennt. Das abgetrennte Beschichtungspolymer wird jedoch nicht rückgeführt, so dass dieses Material verloren geht.

In der WO 00/03797 ist offenbart, dass sich Liposomen und andere biologische Template als Träger für die Herstellung von Nanokapseln durch schichtweise Selbstassemblierung eignen.

Bekannte Verfahren zur Herstellung solcher Strukturen sind die Vernetzung Von Proteinen an Grenzflächen (US 5.498.421) oder an der Oberfläche von Liposomen (Kupcu, S., Sara, M. und Sleytr, U.B., Biochem. Biophys. Acta, 1235 (2): 263-269 (1995)).

In der US 5308701 ist ein Verfahren offenbart, das den Einschluß unter anderem von Liposomen in Mikrokapseln aus Polyelektrolytschichten beschreibt. In der dort beschriebenen Lösung wird jedoch eine Bindung des ersten Polyelektrolyts an die Lipidschicht vermieden. Die Liposomen in der US 5308701 werden vielmehr wie jeder andere gelöste Stoff mit einem Tröpfchen der sie umgebenden Polymerisierung mikroverkapselt. Die Liposomen wirken nicht als Templat der Mikrokapsel, im Ergebnis entstehen deutlich größere Kapseln, die eine Vielzahl von Liposomen in ihrem gelartigen Innern enthalten. Solche Mikrokapseln eignen sich aufgrund ihrer Größe nicht für Anwendungen in der Blutzirkulation.

Die bekannten Grundstrukturen haben weiterhin folgende Nachteile:
- Im US 5.498.421 wird eine Ölphase als Matrix verwendet, die konsequenterweise nur den Einschluß von fettlöslichen Substanzen erlaubt. Das schließt eine Verwendung des Systems für die Mehrzahl der Biopolymere aus.
- Die von Kupcu et al. verwendeten S-Layer-Proteine sind als hochimmunogene Strukturen nicht für den Einsatz bei pharmazeutischen Trägern geeignet.

Nachteilig bei den offenbarten liposomalen Strukturen und Verfahren zu ihrer Herstellung ist weiterhin die mangelhafte Biokompatibilität sowie dass deren Auflösung an extreme Bedingungen wie hohe Temperaturen oder sehr niedrige pH-Werte geknüpft ist.

Die bekannten Verfahren benutzen außerdem überschüssiges Polyelektrolytmaterial, um eine möglichst dichte und reproduzierbare Beschichtung der Oberfläche der Nanokapseln oder Liposomen zu erreichen, deshalb sind weitere Verfahrensschritte notwendig, um das überschüssiges Polyelektrolytmaterial wieder abzutrennen. Weiterhin ist für die Beschichtung von Liposomen mit Polyelektrolyten kein konkretes Verfahren offenbart. Die allgemein vorgeschlagenen Verfahren, so z.B. in WO 00/03797, sind nicht durchführbar, da sich während des Verfahrens nicht auflösbare Aggregate bilden. Ein weiterer Nachteil ist, dass alle Verfahren diskontinuierlich ablaufen, so daß die Nanokapseln oder Liposomen nicht gleichmäßig beschichtet werden können.

Aufgabe der Erfindung war daher die Bereitstellung eines kostengünstigen Verfahrens, das eine einfache Produktion des Templates, insbesondere Liposomen, erlaubt, die mit einer eigenständigen Hüllschicht umgeben sind.

Die vorliegende Erfindung löst dieses technische Problem durch die Bereitstellung von Nano- oder Mikrokapseln mit einem Durchmesser zwischen 20 nm und 40 µm, wobei Templatpartikel in einem wässrigen Medium vorgelegt, mit einem ersten Polyelektrolyten elektrisch umgeladen werden, ohne Trenn- oder Wachschritte mit komplementär geladenen Polyelektrolyten wieder umgeladen werden, und dieser Prozeß mit alternierend geladenen Polyelektrolyten gegebenenfalls weiter fortgesetzt wird. Die alternierende Ladung der Schichten kann dadurch erzeugt werden, daß z.B. die erste Schicht aus anionischen oder überwiegend anionischen Polyelektrolyten und die zweite Schicht aus kationischen oder überwiegen kationischen Polyelektrolyten besteht.

Templats im Sinne der Erfindung sind alle Matritzen, die mit dem erfindungsgemäßen Verfahren beschichtet werden können. Nach Bereitstellung der Matritzen oder Templats können die Partikel z.B. amphiphil beschichtet und in einem weiteren Schritt mit einem Polyelektrolyt beschichtet werden, dass insbesondere eine zur Oberfläche der Teilchenmaterialien entgegengesetzte Ladung aufweist. Zur Bildung von Mehrfachschichten werden die Templats anschließend mit entgegengesetzt geladenen Polyelektrolyten behandelt, d.h., abwechselnd mit kationischen und anionischen Polyelektrolyten. Die Polymerschichten fügen sich auf den vorher geladenen festen Matrizen durch elektrostatische schichtweise Abscheidung von selbst zusammen und bilden so eine mehrschichtige polymere Hülle um die festen Kerne.

Erfindungsgemäß lassen sich solche Strukturen alternierend geladener Polyelektrolyten, z.B. durch Aufbringen von zwei oder mehr wasserlöslichen jeweils komplementär geladenen Polyelektrolytschichten auf der Oberfläche insbesondere von Liposomen herstellen, die zur Ausbildung elektrostatischer Wechselwirkungen befähigt sind. Die direkt aufeinanderfolgenden Polymerelektrolytschichten sind zueinander entgegengesetzt geladen. Eine beliebige Anzahl, mindestens aber zwei solcher Polymerelektrolytschichten, können auf der Oberfläche der Liposomen abgeschieden werden. Polyelektrolyte im Sinne der Erfindung sind Polymere mit ionisch dissoziierbaren Gruppen, die Bestandteil oder Substituent der Polymerkette sein können und deren Zahl so groß ist, daß die Polymeren in der dissoziierten Form wasserlöslich sind. Ist die Konzentration der ionischen Gruppen für eine Wasserlöslichkeit nicht ausreichend, spricht man erfindungsgemäß von Ionomeren. Polymere mit nur einer oder wenigen ionischen Gruppen sind Makroionen, z.B.

Makroanionen oder Makrokationen. Je nach Art der dissoziierbaren Gruppen unterteilt man im Sinne der Erfindung die Polyelektrolyte in Polysäuren und Polybasen. Aus Polysäuren entstehen bei der Dissoziation unter Abspaltung von Protonen Polyanionen, die sowohl anorganisch als auch organisch Polymere sein können. Beispielsweise für Polysäuren, deren Salze als Polysalze bezeichnet werden, sind: Polyphosphorsäure, Polyvinylschwefelsäure, Polyvinylsulfonsäure, Polyvinylphosphonsäure und Polyacrylsäure. Polybasen enthalten als pro-ionische Gruppen solche, die u.a. in der Lage sind, Protonen, z.B. durch Reaktion mit Säuren unter Salz-Bildung, aufzunehmen. Typische Polybasen mit ketten- bzw. seitenständigen dissoziierbaren Gruppen sind Polyethylenimin, Polyvinylamin und Polyvinylpyridin.

Polyelektrolyte, die sowohl anionische als auch kationsiche Gruppen als Substituenten in einem Makromolekül enthalten, sind im Sinne der Erfindung Polyampholyte.

Polyelektrolyte dissoziieren zu Polyionen und den entsprechenden Gegenionen. Sie sind in der wässrigen Lösung ihrer Gegenionen in der Regel gut löslich. Ihre Makromoleküle sind in Lösungen infolge der elektrostatischen Abstoßung der ionischen Gruppen meist linear ausgerichtet; in nicht dissoziierter Form liegen sie dagegen als Knäuelmolekül vor. Mehr- und polyvalente Gegenionen bewirken eine Vernetzung der Polyelektrolyte, die bis zu deren Unlöslichkeit führen kann.

Polyelektrolyte können erfindungsgemäß sowohl Biopolymere, wie z.B. Alginsäure, Gummi arabicum, Nucleinsäuren, Pektine, Proteine u.a., als auch chemisch modifizierte Biopolymere, z.B. Carboxymethylcellulose, Ligninsulfonate und sythetische Polymere, z.B. Poly(meth)acrylsäure, Polyvinylsulfonsäure, Polyvinylphosphonsäure, Polyethylenimin sein. Weitere Polyelektrolyte sind dem Fachmann z.B. aus der WO 00/28972 oder WO 00/03797 bekannt, die in den Offenbarungsgehalt der Erfindung mit aufgenommen sind.

Es können z.B. solche Polymere zum Aufbau der Polyelektroschicht verwendet, die nicht nur strukturbildend, sondern auch aktivitätstragend sind. Solche Hüllen können zum Beispiel Bindungseigenschaften für andere Moleküle oder katalytische Eigenschaften besitzen. Unter den Proteinen finden sich z.B. solche Polymere mit strukturbildenden und aktivitätstragenden Eigenschaften, so kann beispielsweise Hämoglobin zum Aufbau der Hüllstruktur erfindungsgemäß genutzt werden. Die erfindungsgemäßen Nanokapseln können dann als Blutersatz verwendet werden. Es ist jedoch auch möglich, in die Polyelektrolytschicht Proteine zu integrieren, die vorkommende Merkmale anderer Proteine erkennen und binden können. Geeignete Proteine für diesen Zweck sind insbesondere Lektine, biotinbindende oder antikörperbindende Proteine. Derartige Nanokapseln können die Glykosylierungen, antigene Epitope oder Biotingruppen auf Proteinen oder anderen Makromolekülen erkennen und diese Komponenten hochspezifisch binden.

Als Ausgangsmaterial können Liposomen oder Templatpartikel verwendet werden, deren Größe die der entstehenden Nanokapseln bestimmen. Geeignete Methoden zur Herstellung solcher Liposomen sind dem Fachmann bekannt. Erfindungsgemäß müssen die verwendeten Liposomen insbesondere die Bindung des wasserlöslichen Polymers ermöglichen. Methoden für die kovalente Koppelung in wässrigen Medien sind dem Fachmann bekannt und beinhalten unter anderem die heterofunktionelle und homofunktionelle Verknüpfung von Amino-, Thiol-, Hydraxo-, Hydroxo-, Acidwasserstoff-, Aldehyd-, Carboxylgruppen oder von deren aktivierten Estern in geeigneten Kombinationen.

Die Interaktion zwischen dem Liposom und dem erstem Polyelektrolyten kann sich aufgrund des Charakters der Lipidschicht von den bevorzugten elektrostatischen Interaktionen zwischen den darauffolgenden Schichten unterschieden. Eine mögliche Ausgestaltung der erfindungsgemäßen Lehre beinhaltet daher die Verwendung solcher amphipatischer Polymere oder Polyelektrolyten, die in Verbindung mit einer Lipidschicht ein geladenes Partikel ergeben. Beispiele für solche Polyelektrolyte sind integrale oder membranständige Proteine, amphipatische Polymere wie Alkylacrylate, alkylmodifizierte Zuckerpolymere und andere dieser Stoffe, sie natürlichen oder synthetischen oder halbsynthetischen Ursprungs.

In einem folgenden Schritt wird auf die mit dem ersten Polymer beschichteten Templatpartikel eine zweite Schicht mit einem anderen Polymer aufgebracht. Die Ladungen von erstem und zweitem Polymer sind dabei zueinander komplementär. Erstes und zweites Polymer bilden an der Oberfläche der Liposomen ein Netzwerk aus.

Die verwendeten Polymere besitzen ein Zetapotential, das unter den Bedingungen der Reaktion verschieden von Null ist. Wichtige Größen zur Beeinflussung dieses Potentials sind der pH-Wert der Lösung und die Ionenstärke. Zu den geeigneten Verbindungen zählen eine Vielzahl von Polyelektrolyten, aber auch andere wasserlösliche Polymere mit hinreichend polaren Gruppen. Zu den geeigneten Verbindungen gehören z.B.: Polysaccharide wie Alginsäure, Chitosan, Pektin, Hyaluronsäure, Polymannuronsäure, Polygalacturonsäure, Heparin, Gummi Arabicum, Karajagummi, Xanthangummi, Karragenan, Locus Bean Gum und die Salze dieser Verbindungen sowie carboxylierte, aminierte, hydrazylierte Dextrane, Stärken, Levane, Inuline oder Agarosen.

Weitere Verbindungen sind natürliche oder synthetische Proteine oder Peptide oder andere Homo- oder Heteropolymere aus Aminosäuren, Oligonukleotide, DNA oder RNA in einsträngiger oder doppelsträngiger Form und in linearer oder zirkulär geschlossener Form, synthetische Polymere, wie Polyacrylsäuren, Polyacrylamide, Polyacrylsäureester, und andere Polymere aus Derivaten der Acrylsäure, Polyvinylpyrrolidone, Polyethylenimine, Polystyrensulfonsäuren, Polyallylamine, Polyphosphazene und andere mehr. Weitere Verbindungen sind Hetero- oder Blockpolymere aus den oben zugrundeliegenden Monomeren. Es gehören weiterhin dazu Mischformen der aufgeführten Verbindungen wie glykosylierte Proteine, posttranslational modifizierte Proteine, Proteinkomplexe mit anderen Naturstoffen, Komplexe aus Proteinen und Nukleinsäuren, Kopolymere aus Zuckern und Acrylaten und verwandte Verbindungen, insofern als alle diese Verbindungen insbesondere wasserlöslich sein sollten.

Nach zwei oder mehreren Beschichtungen erhält man Polyelektrolyt-beschichtete Nanokapseln, bei denen insbesondere eine Lipidmembran mit einer äußeren Hülle umgeben ist. Daher sind Nanokapseln nach Anspruch 17 mit Liposamen oder Emulsionen als Templatpartikel mit mehr als zwei Polyelektrolytschichten Gegenstand der Erfindung. Diese Hülle verändert vorteilhafterweise die Oberflächeneigenschaften der Liposomen und erhöht deren Stabilität. Sie kann z. B. durch Einwirkung chemischer Quervernetzer weiter verfestigt werden.

Da die Beschichtungsreaktion nach dem erfindungsgemäßen Verfahren insbesondere sehr schnell geführt werden kann, können chemische Quervernetzer vorteilhafterweise bereits zu Beginn der Reaktion der Suspension zugemischt werden und zweckmäßigerweise erst nach dem Ende der Beschichtungsreaktion aus der Suspension abgetrennt werden, wenn dies für die nachfolgende Verwendung wesentlich ist.

Das Verfahren erlaubt es, dass die Beschichtungsreaktion insbesondere innerhalb von wenigen Sekunden abgeschlossen sein kann, und selten länger als wenige Minuten dauert.

Geeignete Vernetzer sind alle Verbindungen, die z.B. in der WO 00/28972 angegeben sind. Insbesondere Vernetzer, die die elektrische Nettoladung der Polyelektrolyte nicht oder nicht wesentlich beeinflussen.

Die verwendeten Liposomen müssen insbesondere die Bindung es ersten wasserlöslichen Polymers ermöglichen. Geeignete Komponenten zur Erzeugung solcher Liposomen sind geladene amphipatische Verbindungen, die sich in die Lipidschicht einlagern können, vor allem ohne diese zu zerstören. Zu den geeigneten Verbindungen gehören natürliche oder synthetische Phospholipide und deren Derivate, insbesondere Phosphatidylserin, Phospatidylinositol, Phosphatidylglycerol oder Phosphatidsäure, aber auch Sphingolipide, Ceramide, Tetraetherlipide oder andere Etherlipide sowie geladene Derivate des Cholesterols wie Cholesterolsulfat, Cholesterolhemisuccinat, Dimethylaminoethylcarbamoyl-Cholesterol und andere dieser Verbindungen. Zu den geeigneten Verbindungen gehören weiterhin Alkylcarbonsäuren, Alkylsulfonsäuren, Alkylamine, Alkylammoniumsalze, Dialkylamine oder -ammoniumverbindungen wie DOTAP oder DOTIM, Phosphorsäureester mit langkettigen Alkoholen und weitere membranbildende oder membranständige geladene Verbindungen. Ungeladene Membranbestandteile wie Phosphatidylcholin, Phosphatidylethanolamin, α-Tocopherol, Cholesterol und andere mehr können zusätzlich als membranbildende Komponenten verwendet werden.

Die Liposomen als auch die verwendeten Polymere besitzen insbesondere eine Vielzahl von Ladungen, die letztlich zu einer Bindung der Komponenten aneinander und zu den erfindungsgemäßen Nanokapseln führen können.

Die Liposomen können uni- oder multilamellare Membranstrukturen besitzen. Bevorzugt sind uni- oder oligolamellare Liposomen mit einer Größe zwischen 20 und 1000 nm, bevorzugt zwischen 50 und 500 nm, besonders bevorzugt zwischen 70 und 300 nm.

Mit Vorteil ist es möglich, dass sich durch die schnelle Abfolge der einzelnen Mischprozesse die Aggregationsneigung der Mischungen verringert, so dass auch Beschichtungen bei höheren Lipid- und Polymerkonzentrationen möglich sind, wobei insbesondere die Integrität der Liposomen im kontinuierlichen Beschichtungsverfahren besser erhalten bleibt als beispielsweise beim diskontinuierlichen Rührprozeß.

In einer bevorzugten Ausführungsform ist die Mischkammer ein statischer Mikromischer, der zu einer besonders schnellen und gleichmäßigen Mischung auch geringer Flüssigkeitsströme führt. Geeignete Mischer sind in der DE 199 25 184A1 beschrieben.

Diese Ausführungsvariante des Verfahrens funktioniert weitgehend unabhängig von der Natur des zur Beschichtung verwendeten Liposoms oder Templates. Die Vorteile der schnellen, mengenoptimierten und zwischenschrittfreien Herstellung von Nanokapseln lassen sich auch mit den bisher beschriebenen kolloidalen Liposomen oder Templaten nutzen. Vorteilhaft kann das Verfahren bei der Herstellung von Polyelektrolythüllen auf instabilen Templaten angewendet werden. So lassen sich z.B. mit dem Verfahren auch Tröpfchen einer Öl-in-Wasser-Emulsion stabilisieren.

In einer weiteren bevorzugten Ausführungsvariante der Erfindung werden die Liposomen nach der Beschichtung mit Polyelektrolyten aufgelöst, vorzugsweise durch Auswaschen mit einem Detergenz. So können Strukturen in Form von Hohlkugeln entstehen, bei denen die Liposomen nach der Vernetzung aufgelöst wurden. Dabei kann es z.B. zur Freisetzung von solchen Polymeren, die lediglich an der Lipidschicht, nicht aber untereinander gebunden sind, sowie zum Zerfall nicht hinreichend vernetzter Strukturen kommen. Die Nanokapseln lassen sich von diesen Zerfallsprodukten durch Sedimentation, Gelfiltration oder Ultrafiltration abtrennen.

Geeignete Detergenzien zur Auflösung der innenliegenden Liposomen sind alkylierte Zucker wie etwa Octylglucosid, Salze der Cholsäure und ihrer Derivate, Alkylsulfonsäuren, Polyoxyethylensorbitole oder ähnliche Verbindungen. Die Nanokapseln im Nanometerbereich im Sinne dieser Erfindung bestehen dann nur aus einem Polymergerüst, dass die Oberfläche einer Kugel einnimmt. Die formgebenden Liposomen können insbesondere so entfernt werden, dass die Größe der entstandenen Hohlkugeln durch die verwendeten Liposomen bestimmt ist.

Die Permeabilität der Hüllschicht der Nanokapseln kann vorteilhafterweise durch das Auswaschen der Liposomen wesentlich erhöht werden. Dieser Prozeß beinhaltet beispielsweise die Passage von Detergensmolekülen und Mischmicellen durch die äußere Hüllschicht. In gleicher Weise können Substrate und Produkte einer im Innern der Hohlkugel stattfindenden Reaktion ausgetauscht werden. Eine Anordnung zur Durchführung solcher Reaktionen besteht vorzugsweise aus Hohlkugeln mit im Inneren befindlichen enzymatisch aktiven Stoffen mit hohem Molekulargewicht, deren Liposomen durch Detergenzien ausgewaschen wurden. Geeignete Stoffe für einen solchen Einschluß sind insbesondere Enzyme oder Ribozyme. Es ist jedoch auch möglich, lediglich die nicht gebundenen Polymere auszuwaschen, so daß die Lipidschicht erhalten bleibt. So können nur solche Stoffe ausgetauscht werden, die durch die Lipidschicht diffundieren. Das sind amphiphile Moleküle, wie beispielsweise Phenylalanin. Nanokapseln, die Phenylalanin-4-Hydroxylase oder Phenylalanin-Ammoniak-Lyase enthalten, können insbesondere zum Abbau bestimmter Aminosäuren bei Phenylketonurie eingesetzt werden.

In einer weiteren bevorzugten Ausführungsvariante der Erfindung wird die Hüllschicht aus Polyelektrolyten nach ihrer Abscheidung mit bifunktionellen Reagenzien kovalent vernetzt.

In einer weiteren vorteilhaften Ausführungsvariante des Verfahrens werden als Polyelektrolyte natürliche oder synthetische Polymere oder Mischformen dieser Verbindungen verwendet, beispielsweise Polysäuren oder Polybasen. Polysäuren werden unter anderem bei der Dissoziation von Polyanionen gebildet, wobei die Polyanionen anorganische wie auch organische Polymere sein können. Polybasen enthalten Gruppen, die insbesondere in der Lage sind, Protonen aufzunehmen.

In einer weiteren Ausführungsvariante der Erfindung umfassen die Polyelektrolyte Alginsäuren, Chitosan, Nucleinsäuren, Polynucleotide und/oder Proteine, vorzugsweise Albumin, Hämoglobin, Myoglobin, Antikörper, Proteasen, α2-Makroglobulin, Fibronectin, Collagen, Vitronectin, Protein A, Protein G, Avidin, Streptavidin, Concanavalin A und/oder Wheat Germ Agglutinin.

In einer weiteren Ausführungsvariante des Verfahrens ist vorgesehen, daß in die Nanokapseln Wirkstoffe eingeschlossen werden. Wirkstoffe können beispielsweise biologisch oder chemisch aktive Verbindungen sein, die in geringen Konzentrationen chemische, biochemische, biophysikalische und physiologische Prozesse, z.B. Stoffwechselprozesse, in Lebewesen qualitativ oder quantitativ so beeinflussen, daß eine Aktivierung oder Hemmung bestimmter Prozesse erfolgt. Hierbei können beispielsweise Wirkstoffe eingesetzt werden, die natürlicherweise innerhalb von Organismen vorkommen, wie beispielsweise Vitamine oder Hormone; es ist jedoch auch möglich, körperfremde Wirkstoffe einzusetzen, wie z.B. Biozide.

In einer weiteren Ausführungsvariante des Verfahrens ist vorgesehen, daß die Liposomen Phophatidylserin, Phosphatidylglycerol, Phosphatidsäure, Sphingolipide, Ceramide, Tetraetherlipide, Cholesterolsulfat, Cholesterolhemisuccinat, Dimethylaminoethylcarbamoyl-Cholesterol, Alkylcarbonsäure, Alkylsulfonsäuren, Alkylamine, Alkylammoniumsalze, Dialkylamine, DOTAP, DOTIM, Phosphorsäureester mit langkettigen Alkoholen, Phosphatidylcholin, Phosphatidylethanolamin und/oder α-Tocopherol umfassen.

In einer weiteren Ausführungsvariante der Erfindung wird die Beschichtung mit den Polymeren bei einer Lipidkonzentration kleiner 2 mM. durchgeführt. Bevorzugt sind Lipidkonzentrationen kleiner 1 mM, besonders bevorzugt kleiner 0,5 mM und ganz besonders bevorzugt kleiner 0,2 mM. Durch die gewählten Verdünnungen ist es vorteilhafterweise möglich, die Aggregationsbildung zu unterdrücken.

In einer weiteren Ausführungsform ist vorgesehen, daß bei dem Verfahren Liposomen mit 10 bis 50 Mol %, bevorzugt 30 bis 50 Mol % und insbesondere 35 bis 45 Mol % geladenen Sterolen verwendet werden.

Zweckmäßig ist es bei dem Einsatz von Phospholipiden mehr als 10 Mol%, bevorzugt mehr als 40 Mol% und besonders bevorzugt mehr als 60 Mol% zu verwenden.

Mit Vorteil lässt die Menge des abgeschiedenen Polymers und damit die Dichte der erzeugten Schichten mit der Dichte der Ladungsträger auf dem Liposomen steuern. Dieser Befund ist insofern überraschend, als die Ladungsträger in der liposomalen Membran beweglich sein können und relativ geringe Anteile schon zur stöchiometrischen Absättigung des Polymers ausreichen. Wenn die Ladungsträger selbst membranbildende Substanzen sind, wie etwa geladene Phospholipide oder deren Derivate oder auch geladene Dialkyle wie DOTAP oder DOTIM, so können mit Vorteil noch höhere Mengen an Ladungsträgern verwendet werden. In diesem Fall werden bevorzugt Anteile zwischen 10 und 100% des Gesamtlipids verwendet, weiter bevorzugt Anteile zwischen 40 und 100% und ganz besonders bevorzugt Anteile zwischen 40 und 80% der genannten Substanzen. Eine weitere Erhöhung der Ladungsträgerdichte an der Oberfläche ist vorteilhafterweise durch die Verwendung mehrfach geladener Gruppen möglich, etwa durch hohe Anteile der zweifach negativ geladenen Phosphatidsäure oder durch mehrfach geladen substituierte membranständige Verbindungen, etwa Konjugaten aus Spermin und Sterolen oder solchen aus Oligopeptiden und Phospholipiden oder solchen aus Heparin und Lipiden oder auch solchen aus anderen multifunktionellen Verbindungen, etwa Oligo- und Polycarbonsäuren oder Oligo- bzw. Polyaminen, und Lipiden. Der Übergang zu Lipid-Polymerkonjugaten ist fliessend und die genannten Beispiele können durch den Fachmann leicht weiter ergänzt werden.

In einer weiteren Ausführungsvariante der Erfindung wird die Beschichtung mit den Polymeren bei einer Salzkonzentration größer 50 mM durchgeführt.

Vorteilhafterweise kann die Dichte der liposomalen Ladungsträger durch die maximale Salzkonzentration der Lösung bestimmt werden, bei der noch eine vollständige Bindung des Polymers an die Template erfolgt. Eine Durchführung der Beschichtung bei einer möglichst hohen Salzkonzentration ist aus zwei Gründen vorteilhaft:
(i) Salzkonzentrationen größer als 50mM führen zu einer deutlichen Kompaktierung hochgeladener Polymere, da die intramolekulare Abstossung verringert wird. Dadurch ist eine dichtere Packung der Polyelektrolyte auf der Oberfläche möglich.
(ii) Eine nachträgliche Erhöhung der Salzkonzentration des Mediums führt nicht immer, aber in vielen Fällen zu einer Aggregation der Partikel, wohl durch partielle Destabilisierung der äußeren Polyelektrolytschicht. Eine Verringerung der Salzkonzentration ist jedoch unschädlich.

Insbesondere benötigt die Beschichtungsreaktion auch bei den Verdünnungen erheblich weniger Zeit, als nach dem Stand der Technik zu erwarten gewesen wäre. Dieser schnelle Reaktionsverlauf ermöglicht den sinnvollen Aufbau eines kontinuierlichen Verfahrens.

Es wurde in diesem Zusammenhang weiterhin überraschend festgestellt, dass die Phasenübergangstemperatur der liposomalen Membran Einfluss auf die Reaktionsgeschwindigkeit hat. So verlaufen die Beschichtungsreaktionen schneller, wenn die Lipidmembran oberhalb der Phasenübergangstemperatur beschichtet wird.

In einer bevorzugten Ausführungsvariante der Erfindung ist vorgesehen, dass ein Reaktionszyklus in weniger als 20 Minuten, bevorzugt in weniger als fünf Minuten, besonders bevorzugt in weniger als einer Minute durchlaufen wird.

In einer weiteren vorteilhaften Ausführungsvariante der Erfindung wird bei der Beschichtungsreaktion oder nach deren Abschluß ein chemischer Vernetzer zugesetzt.

In einer weiteren bevorzugten Ausführungsvariante der Erfindung ist vorgesehen, dass die Templatpartikel eine Größe zwischen 20 nm und 1000 nm, bevorzugt zwischen 50 nm und 500 nm und besonders bevorzugt zwischen 70 nm und 30 nm besitzen.

In einer weiteren bevorzugten Ausführungsvariante der Erfindung werden in einer Schicht zwei oder mehr voneinander verschiedene Polyelektrolyte gleichzeitig oder nacheinander aufgebracht.

In einer bevorzugten Ausführungsvariante sind die Templatpartikel Liposomen.

Es kann zweckmäßig sein, dass die Templatpartikel in einer Öl-in-Wasser-Emulsion vorliegen. Insbesondere kann es zweckmäßig sein, dass die Emulsionen in ihrer Ölphase Wirkstoffe enthalten.

Bei einer bevorzugten Ausführungsvariante der Erfindung weisen die Nanokapseln zuätzlich eine Lipidschicht auf, auf der sich die Polyelektrolytschichten befinden. Die Lipidschicht kann beispielsweise die äußere Ölschicht von Liposomen sein, die sich in der Nanokapsel befinden.

Die Erfindung betrifft auch Strukturen gemäß Anspruch 17, die im Inneren Lipidschichten enthalten, wobei im Inneren der Strukturen eine Flüssigphase vorliegen kann. Prinzipiell ist es möglich, daß die Nanokapseln jede Flüssigkeit, zu der im Sinne der Erfindung auch Suspensionen gehören, in ihrem Inneren enthalten können. Beispiele für Flüssigkeiten sind beispielsweise Wasser, Puffer, Flüssig-Aerosole und andere.

Es kann zweckmäßig sein, wenn die Strukturen in ihrem Inneren eine nicht wassermischbare Ölphase enthalten.

In einer weiteren Ausführungsvariante der Erfindung befinden sich in den Strukturen Wirkstoffe. Wirkstoffe im Sinne der Erfindung sind Stoffe, die - in relativ kleinen Mengen vorkommend oder zugeführt - eine physiologische Wirkung entfalten können. Beispiele für derartige Wirkstoffe sind Hormone, Vitamine, Enzyme, Spurenelemente, Pharmaka, Futterzusätze, Düngemittel, Schädlingsbekämpfungsmittel und andere.

Die Wirkstoffe im Sinne der Erfindung können unter anderem biochemische und physiologische Prozesse in Lebewesen qualitativ oder quantitativ im Sinne einer Aktivierung oder Hemmung beeinflussen.

In einer weiteren bevorzugten Ausführungsform der Erfindung sind die Wirkstoffe Bestandteil der Polyelektrolytschicht oder der Lipidschicht der Strukturen. Vorteilhafterweise können so beispielsweise lipidlösliche Komponenten, wie beispielsweise fettlösliche Vitamine, mit hohen Konzentrationen in die Nanokapseln eingebracht werden.

In einer weiteren bevorzugten Ausführungsform der Erfindung ist der Wirkstoff ein Katalysator, ein Biokatalysator, Pharmaka, ein Enzym, ein pharmazeutischer Stoff, ein Protein, ein Peptid, ein Oligonucleotid, ein Sensor, Nucleinsäuren und/oder ein Kristall. Die Wirkstoffe können beispielsweise in die Nanokapseln eingeschlossen werden oder wenn sich innerhalb der Nanokapseln Liposomen befinden, können die genannten Wirkstoffe auch in die Liposomen eingeschlossen werden. In diesem Falle können Liposomen verwendet werden, welche die einzuschließenden Stoffe bereits enthalten. Methoden zur Herstellung solcher Liposomen sind dem Fachmann bekannt. Die verwendbaren Stoffe sind insofern spezifiziert als sie die Integrität der Liposomen nicht nachteilig beeinflussen sollten, wie etwa Detergenzien. Geeignete Stoffe sind z.B. Proteine, Peptide, Vitamine, Hormone, Kohlenhydrate oder Nukleinsäuren sowie Gemische derselben. Zu den geeigneten Stoffen gehören weiterhin Antibiotika, Fungizide und antivirale Agenzien, Antikörper, Zytostatika und Immunsuppressiva, Analgetika, Anästhetika, Antidepressiva, Antidiabetika, Antihypertensika, Antikoagulatien, antiinflammatorische, angstlösende, sedative, antiarrhythmische, antiarthritische Wirkstoffe, Bronchodilatoren, hypoglykämische und hypolipidämische Wirkstoffe sowie Wirkstoffe zur Stimulierung der Erythropoese und apoptoseauslösende Stoffe. Für den Einschluß von Cargomolekülen kann man ebenfalls von Liposomen ausgehen, die diese Stoffe bereits enthalten oder an denen solche Stoffe gebunden sind. Die eingeschlossenen oder gebundenen Stoffe verbleiben bei allen Reaktionsschritten in den innen liegenden Liposomen oder in der Lipidschicht.

Die Erfindung betrifft auch die Verwendung der erfindungsgemäßen Nanokapseln als Container oder Transporter bei pharmazeutischen Zubereitungen.

Die Verwendung der beschichteten Liposomen erfolgt insbesondere als Container und Transporter für biologisch wirksame Stoffe.

Aufgrund der Vielzahl von verwendbaren Komponenten lassen sich die beschichteten Liposomen und die lipidfreien Nanokapseln für eine große Zahl von Anwendungen einsetzen. Die Verwendung der Nanokapseln erweitert das Spektrum der Trägermaterialien im Sinne eines drug targeting, eines Transfervektors, einer Depotform oder für eine Enzymersatztherapie. Dabei können die verwendeten Komponenten vorteilhafterweise sowohl strukturbildend als auch aktivitätstragend sein. Die beschichteten Liposomen und die lipidfreien Nanokapseln lassen sich insbesondere aus Stoffen mit einer antigenen Wirkung herstellen oder aus solchen, die keine Immunantwort hervorrufen.

Die Verwendung der Nanokapseln wird möglich durch die Vorteile des erfindungsgemäßen Verfahrens, das erstmals die Vorteile des schonenden Einschlusses von Wirkstoffen in Liposomen mit einer effizienten Technologie zur Beschichtung von kolloidalen Partikeln kombiniert, die ohne den Einsatz von weiteren Hilfsstoffen ausgeführt werden kann und daher von besondere Vorteile bei pharmazeutischen Verwendungen erlaubt.

Für die Verwendung in Detektionssystemen sind enzymatische oder fluorescierende Eigenschaften der Nanokapseln vorteilhaft. Geeignete Stoffe mit solchen Eigenschaften sind das Green Fluorescent Protein oder Phycobiliproteine. Andere geeignete Polymere lassen sich mit fluorescierenden Stoffen modifizieren. Geeignete Methoden dazu sind dem Fachmann an sich bekannt und beinhalten die kovalente Bindung des aktivierten Fluorophors an entsprechende Gruppen des Polymers oder die Komplexbildung von fluorescierenden Metallionen mit chelatisierenden Gruppen des Polymers.

Unter den Proteinen finden sich Polymere mit enzymatischer Aktivität, etwa als Peroxidasen, Phophatasen, Proteasen, Dehydrogenasen, Glucosidasen und andere mehr.

Nanokapseln mit einem solchen Aufbau können aber auch für eine zielgesteuerte Applikation von Arzneistoffen benutzt werden. Zu den hochspezifischen Molekülen gehören daher insbesondere solche, die mit der Oberfläche von Zellen interagieren können. Komplementäre Paare in diesem Sinne sind Antikörper und membranständige Antigene, Lektine oder Selektine und membranständige Antigene, Lektine oder Selektine und membranständige Glykosylierungen, Hormone und deren Rezeptoren und andere mehr. Vorteilhaft ist der modulare Aufbau der Strukturen, der zum einen die Erzeugung einer offenen Anzahl von Spezifitäten auf einigen wenigen Hüllschichten erlaubt, zum anderen einen sehr ökonomischen Einsatz der letztlich spezifitätsbestimmenden Komponenten gestattet. Die Valenz der erhaltenen Struktur, das heißt die Anzahl der oberflächlich gebundenen spezifitätsbestimmenden Komponenten läßt sich leicht durch Titration verändern. Eine hohe Dichte dieser Komponenten ist gleichbedeutend mit einer hohen Avidität und ermöglicht stabile Interaktionen auch bei ungünstigen Bindungskonstanten der einzelnen Wechselwirkung, wie sie etwa zwischen MHC-Komplexen und T-Zell-Rezeptoren gegeben ist.

In einer weiteren vorteilhaften Ausgestaltung der Erfindung werden Nanokapseln nach ihrer Entstehung mit anderen Stoffen modifiziert. Eine wichtige Variante dieser Ausgestaltung ist die Modifizierung der Oberfläche der Nanokapseln mit Polyethylenglykol oder mit Zuckern oder anderen Polyalkoholen. Eine solche Beschichtung führt zu Partikeln mit einer verbesserten Verträglichkeit bei pharmazeutischen Anwendungen. Benutzt man die hier beschriebene Struktur zum Einschluß von Enzymen oder anderen Katalysatoren, so ist durch die diffusionsoffene Architektur eine hohe Verfügbarkeit der eingeschlossenen Aktivität gewährleistet. Bei der gewählten Größe im Mikrometer- und Submikrometerbereich sind die Diffusionswege zudem extrem kurz. Andere Anwendungen sind bei der Herstellung von Mikrokristallen bestimmter Größe auf chemischen oder biochemischen Weg gegeben.

In einer weiteren Verwendung kommen insbesondere solche Stoffe mit enzymatischer Aktivität zum Einsatz, deren Substrate und Produkte durch die Hüllschicht ausgetauscht werden können.

Nanokapseln im Sinne der vorliegenden Erfindung besitzen eine diffusionsoffene Struktur, die den Austausch von Molekülen mit signifikanter Größe, etwa beim Herauslösen der Lipidschicht, zuläßt. Große Moleküle wie etwa Enzyme können jedoch von der Hüllschicht zurückgehalten werden. In weiteren erfindungsgemäßen Verwendungen der Nanokapseln sind diese mit solchen Enzymen gefüllt, die Reaktionen katalysieren, deren Substrate und Produkte die Hüllschicht passieren können. Diese Art der Verpackung eines biologischen Makromoleküls in Nanokapseln hat gegenüber dem Stand der Technik den Vorteil extrem geringer Diffusionswege und einer damit verbundenen Erhöhung der spezifischen Aktivität des eingeschlossenen Enzyms. Darüber hinaus kann die Einwirkung von vernetzenden Agenzien, wie sie bei der chemischen Fixierung auftritt, vermieden werden.

Es ist jedoch auch möglich, signalgebende Systeme, wie etwa Meerrettich-Peroxidase oder alkalische Phosphatase oder fluorescensmarkierte Makromoleküle, in solche Nanokapseln einzuschliessen, die spezifische Bindungseigenschaften gegenüber anderen Stoffen aufweisen. Solche Systeme sind zur Detektion dieser anderen Stoffe geeignet, insbesondere in der medizinischen oder biochemischen Diagnostik. Vorteilhaft gegenüber den Liposomen ist die Tatsache, daß Nanokapseln stabil gegenüber Detergenzien sind, insbesondere auch gegenüber solchen Detergenzien, die zur Unterdrückung unspezifischer Bindungen in solchen Verfahren eingesetzt werden, wie etwa Tween 20 oder Triton X-100.

In einer Variante dieser erfindungsgemäßen Verwendung sind die Nanokapseln selbst Träger des signalgebenden Systems. Vorteilhaft werden Nanokapseln präpariert, deren Polymere fluorescierende Eigenschaften besitzen. Dabei werden fluorescierende Derivate von P1 und/oder P2 zum Aufbau der Nanokapseln verwendet oder die Nanokapseln nach ihrer Herstellung mit fluorescierenden Stoffen kovalent verbunden.

In einer erfindungsgemäßen Verwendung der Nanokapseln sind diese so beschaffen, daß sie spezifisch an Zielzellen von Säugetieren binden. Nanokapseln im Sinne dieser Verwendung besitzen eine oder mehrere Klassen von Liganden auf ihrer Oberfläche, deren komplementäre Bindungspartner sich auf der Oberfläche der Zielzellen befinden. Nanokapseln mit solchen Eigenschaften sind Träger für Therapeutika, die diese an eine definierten Wirkort dirigieren. Die innere Lipidschicht der Hohlkugeln kann bei dieser Verwendung erhalten bleiben, wenn es dem Einschluß der zu transportierenden Substanz dienlich ist.

In einer Variante dieser erfindungsgemäßen Verwendung enthalten die Nanokapseln Stoffe, gegen die eine Immunantwort ausgelöst werden soll.

In einer vorteilhaften Variante dieser Ausgestaltung der Erfindung werden die Nanokapseln zum Transfer von Wirkstoffen in das Cytosol von Säugetierzellen benutzt. Diese Nanokapseln sind so beschaffen, dass sie von Säugetierzellen endozytiert werden. Nanokapseln für diese Ausgestaltung der Erfindung bestehen aus einer Hüllschicht, die von den Hydrolasen des Endosoms abgebaut werden kann. Sie werden darüberhinaus aus solchen Liposomen hergestellt, deren Membran mit der des endozytotischen Vesikels fusionieren kann. Vorteilhaft bei dieser Ausgestaltung der erfinderischen Lehre ist die Tatsache, dass eine solche Fusion nicht zu einer Freisetzung lytischer endosomaler Aktivitäten in das Zellinnere führen kann. Nanokapseln für diesen Verwendungszweck können mit unterschiedlichen Wirkstoffen beladen werden. Der beschriebene Transportweg ist jedoch von besonderem Vorteil beim Transport nicht membrangängiger biologischer Makromoleküle, wie etwa Proteine, Peptide, Antikörper, Enzyme, Oligonukleotide, DNA, RNA, Hormone, aber auch von Antibiotika, Fungiziden und antivirale Agenzien sowie von Zytostatika.

In einer weiteren vorteilhaften Ausführungsvariante der Erfindung ist vorgesehen, die Nanokapseln in der biochemischen Diagnostik einzusetzen.

In einer weiteren vorteilhaften Ausführungsvariante der Erfindung ist weiterhin vorgesehen, die Nanokapseln zur Herstellung von Mikrokristallen, Herbiziden, Pestiziden und/oder Pigmenten zu verwenden. Mikrokristalle im Sinne der Erfindung sind beispielsweise Werkstoffe, die aus einem oder mehreren Materialien bestehen und eine mikroskopische Ordnung aufweisen. Es kann sich beispielsweise um Peptidmoleküle handeln. Herbizide im Sinne der Erfindung sind Substanzen, die in der Lage sind, alle Wild- und Kulturpflanzen, die an ihrem jeweiligen Standort unerwünscht sind, in ihrer Entwicklung negativ zu modifizieren. Es kann sich beispielsweise um Entblätterungsmittel, Krautabtötungsmittel oder andere als Aerosol, Flüssigkeit oder Feststoff vorliegende Substanzen handeln. Die Herbizide in den erfindungsgemäßen Nanokapseln können sowohl in der Vorsaat, dem Vorauflauf und dem Nachauflauf der Kulturpflanzen eingesetzt werden. Die einzelnen Wirkstoffe können dabei so gewählt werden, daß die anmeldungsgemäßen Nanokapseln in den Boden eingebracht werden, bzw. im Blattbereich der Pflanze. Entfalten die Herbizide ihre Wirkung direkt am Benetzungsort, handelt es sich insbesondere um Kontaktherbizide. Im einzelnen können eingesetzt werden: Photosynthesehemmer, Atmungshemmer, Buckstoffhemmer, Keimhemmer, Carotinsynthesehemmer und andere. Pestizide im Sinne der Erfindung sind alle Zubereitungen, die Schadorganismen oder lästige Organismen unschädlich machen, vernichten oder ihrer Einwirkung vorbeugen können. Hierzu können beispielsweise Mittel gegen Fliegen, Bremsen, Mücken, Schaben, Wanzen oder Flöhe und andere gehören, wie auch Erzeugnisse, die gegen Ratten, Mäuse, Käfer oder Motten eingesetzt werden können. Pigmente im Sinne der Erfindung sind ein im wesentlichen unlösliches, anorganisches oder organisches, buntes oder unbuntes Farbmittel.

Die Erfindung betrifft auch eine Vorrichtung zur kontinuierlichen Beschichtung von Liposomen in mehreren Polyelektrolytschichten, wobei in dem von einer Pumpe bewirkten Hauptfluss der Liposomen nach einem Dämpfungsglied für jeden getrennt zugeführten, von einem Pumpe bewirkten Zufluss des aufzubringenden Polyelektrolyts, ein Mischer vorgesehen ist, wobei zwischen den Mischern jeweils ein Zeitglied angeordnet ist und zwischen den Pumpen für das Einbringen der Polyelektrolyte und den Mischern jeweils ein Dämpfungsglied angeordnet ist.

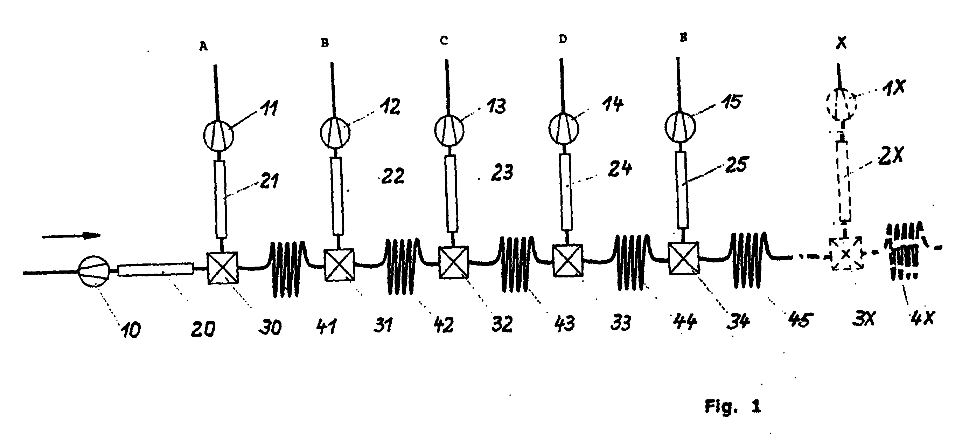

Bestandteil der erfindungsgemäßen Lehre ist demnach eine Apparatur zur kontinuierlichen Beschichtung von Liposomen mit mehreren Polyelektrolythüllen. Dabei wird mit einer Pumpe ein konstanter Fluß der Liposomenlösung in einem Schlauchsystem ermöglicht. Die zur Beschichtung benutzten Polyelektrolyte werden nacheinander in das Schlauchsystem eingeführt, wie in der Figur 1 gezeigt. Die Mischung der Reaktanden erfolgt durch hohen Fluß oder mit dynamischen oder statischen Mischern an den Zuführungspunkten. Das Volumen der Schlauchabschnitte zwischen den einzelnen Miechpunkten ist dabei so bemessen, dass bei der gegebenen Fließgeschwindigkeit eine hinreichend lange Reaktionszeit bis zur Erreichung des nächsten Mischpunktes zur Verfügung steht.

Die Vorrichtung kann in einer besonderen Ausführungsform so ausgebildet sein, dass die Schlauchleitung das Zeitglied bildet.

Liposomen oder Emulsionen gemäß der hier vorliegenden Erfindung weisen mehrere vorteile auf und sind hydrophile, permeable und detergensstabile Strukturen aus vernetzten Polymeren, die sich aufgrund der Vielzahl von verwendbaren Komponenten für eine große Zahl von Anwendungen spezifizieren lassen. Die vorliegende Erfindung erweitert erheblich das Spektrum solcher Stoffe, die sich als Trägermaterialien im sinne eines drug targeting, eines Transfervektors, einer Depotform oder für eine Enzymersatztherapie verwenden lassen. Dabei können die verwendeten Komponenten sowohl strukturbildend als auch aktivitätstragend sein. Die beschriebenen Hohlkugeln lassen sich aus Stoffen mit einer antigenen Wirkung herstellen oder aus solchen, die keine Immunantwort hervorrufen.

Bei dem anmeldungsgemäßen Kontinuierlichen Verfahren, welches zahlreiche vorteile gegenüber dem Stand der Technik aufweist, wurde überraschend gefunden, daß mit geeigneten Masseverhältnissen eine praktisch vollständige Bindung des eingesetzten Polymers an die Liposomen möglich ist, so dass Trennungsschritte zwischen den einzelnen Beschichtungen entfallen können. Dieser Umstand trägt entscheidend zur Prozeßökonomie des Verfahrens bei. Die geeignete Menge des jeweils benötigten Schichtmaterials entspricht in etwa der maximal unter den gegebenen Reaktionsbedingungen gebundenen Menge und ist daraus ableitbar.

Zur Feststellung der für das jeweilige Partikel geeigneten Menge an Polyelektrolyt titriert man in mehreren kleinen Ansätzen steigende Mengen Polymer zu vorgelegter Partikelsuspension und bestimmt anschließend die Größe der Partikel. Die Größe der Liposomen nimmt durch Aggegratbildung mit dem Polymer schnell zu und überschreitet dann ein Maximum. Erreicht die Größe mit steigenden Polymermengen wieder den ursprünglichen Wert, dann wird die optimal zur Beschichtung geeignete Menge verwendet.

Überraschenderweise wurde bei einer Reihe von Polymeren, insbesondere bei Proteinen, eine geringe Neigung zur Bildung von Aggregaten festgestellt. Mit diesen Stoffen können Partikel beliebiger Beschichtungsstufen, also Templatpartikel oder bereits beschichtete Spezies zunächst so beschichtet werden, dass es noch zu keiner Umladung kommt. Diese wird dann durch weitere Zugaben von gleichem oder von gleichgeladenem, aber stofflich verschiedenem Polyelektrolyt erreicht. Auf diese Weise lassen sich einzelne Schichten etwas mit aktivitätstragenden Molekülen dotieren. Die dosierte Verwendung von spezifisch bindenden Komponenten ermöglicht eine Variierung der Bindungsstärke des Partikels.

Eine wichtige Variante der erfinderischen Lehre besteht darin, dass Liposomen schon im Prozess ihrer Entstehung mit komplementär geladenen Polyelektrolyten in Kontakt gebracht werden. Solche Liposomen enthalten sowohl eingeschlossene als auch außen anhaftende Polyeletrolytmoleküle. Die Aggregationsneigung verringert sich mit der Konzentration des Polyelektrolyts, bevorzugt werden weniger als 500 µm/mg Lipid, besonders bevorzugt weniger als 150µg Protein/mg Lipid verwendet.

In verdünnter Suspension nach den oben beschriebenen bevorzugten Konzentrationen sind solche Partikel für mehrere Minuten bis Stunden stabil und können nach dem erfindungsgemäßen Verfahren weiter beschichtet werden. Vorteilhaft wirkt sich hier aus, dass auch beim Einschluß von Wirkstoffen keine Trenn- oder Waschschritte notwendig sind.

Weiterhin wurde überraschend festgestellt, daß sich die Menge des abgeschiedenen Polymers und damit die Dichte der erzeugten Schichten mit der Dichte der Ladungsträger auf den Liposomen steuern lässt.

Ein weiterer Vorteil gegenüber dem Stand der Technik ist, daß sich der unerwünschte Prozess der Aggregatbildung durch sehr schnelles Mischen der Reaktionspartner und geeignete Verdünnungen unterdrücken lässt. Ein weiterer Vorteil ist, daß die Dichte der liposomalen Ladungsträger durch die maximale Salzkonzentration der Lösung bestimmt werden kann.

Bei bekannten Verfahren tritt bei der Herstellung solcher Gemische starke Aggregatbildung auf, die zur Flockung der Reaktionspartner führt. Vorteilhafter lässt sich dieser unerwünschte Prozeß durch sehr schnelles Mischen der Reaktionspartner und geeignete Verdünnungen und dem unmittelbar aufeinanderfolgen der einzelnen Schritte unterdrücken läßt.

Im folgenden soll die Erfindung anhand von Beispielen näher erläutert werden, ohne dass die Erfindung auf diese Beispiele zu beschränken ist.

### Beispiele

### Verwendete Abkürzungen

- CTAB: Cetyltrimethylammoniumbromid
- FITC: Fluoresceinisothiocyanat
- PC: Phosphatidylcholin
- MES: 2-(N-Morpholino)-ethansulfonsäure
- PSS: Polystyrensulfonsäure
- PEI: Polyethylenimin
- DPPC: Dipalmitoylphosphatidylcholin
- DPPG: Dipalmitoylphosphatidylglycerol
- DOPE: Dioleoylphosphatidylethanolamin
- CHEMS: Cholesterinhemisuccinat
- Hepes: N-(2-Hydroxyethyl)piperazin-N'-(2-ethansulfonsäure)
- PLL: Poly-L-Lysin
- PAS: Polyacrylsäure
- BSA: Rinderserumalbumin

### Beispiel 1

### Nanokapseln aus Polystyrensulfonsäure und Polyethylenimin

### Herstellung der Liposomen

400 mg PC aus Soja und 9,7 mg CTAB werden in Ethanol gelöst und unter Vakuum bis zur Trockne eingedampft. Der Lipidfilm wird anschließend mit einem Puffer (10 mM MES, 150 mM NaCl pH 6.5) rehydratisiert. Die Suspension wird dann mehrmals durch isopore Polykarbonatmembranen mit einer Porengröße von 0,2 µm gedrückt.

### Beschichtung mit PSS

PSS (Mr 70000) wird mit einer Konzentration von 10 µg/ml in MES-Puffer (10 mM pH 6.5) gelöst. Die Liposomen werden mit dem gleichen Puffer so verdünnt, dass eine Lipidkonzentration von 200 µg/ml erreicht wird. Gleiche Volumina beider Lösungen werden unter Rühren zusammengegeben. Anschließend wird die Suspension mittels Tangentialdialyse aufkonzentriert.

### Beschichtung mit PEI

PEI (Mr 60000) wird mit einer Konzentration von 5 µg/ml in MES-Puffer (10 mM pH 6.5) gelöst. Die mit PSS beschichteten Liposomen werden im gleichen Puffer soweit verdünnt, dass eine Lipidkonzentration von 200 µg/ml erreicht wird. Gleiche Volumina beider Lösungen werden unter Rühren zusammengegeben. Anschließend wird die Suspension mittels Tangentialdialyse aufkonzentriert.

Weitere Beschichtungen können wie in den beiden oberen Schritten aufgebracht werden. Die eingesetzte Menge Polymer bindet hinreichend vollständig an die Partikeloberfläche, so dass keine Reinigungsschritte zwischengeschaltet werden müssen.

### Beispiel 2

### Analyse der entstandenen Strukturen

Die Intensität des von einer Partikelsuspension erzeugten Streulichts wird in einer Apparatur zur dynamischen Lichtstreuung gemessen. Nach Zugabe von Detergenz sinkt die gemessene Intensität bei Liposomen auf weniger als 5% des Ausgangswertes. Nach Beschichtung mit drei oder mehr Polymerschichten bleiben mehr als 40% der Intensität erhalten.

Die Stabilität der so erhaltenen, liposomenfreien Hohlkugeln wird durch Zugabe von NaCl geprüft. Die Partikel sind mindestens gegenüber 1 M NaCl beständig.

### Beispiel 3

### Stabilität der Strukturen im Serum

Die beschichteten Liposomen aus Beispiel 1 werden durch Ultrafiltration aufkonzentriert, so dass die Lipidkonzentration bei 1 mg/ml liegt und dann mit der gleichen Menge humanem Serum gemischt. Die Intensität des von der Partikelsuspension gestreuten Lichts wird in einer Apparatur zur dynamischen Lichtstreuung gemessen. Gleichzeitig wird die Größe der Partikel bestimmt. 24 h nach der Zugabe des Serums sind mehr als 90% der Partikel in ihrer urspünglichen Größe noch nachweisbar.

### Beispiel 4

### Herstellung fluorescierender Nanokapseln

### Modifizierung von PEI

100 mg PEI werden in 10 ml Boratpuffer (0.1 M pH 9.0) gelöst und mit 1ml Fluorescein-Isothiocyanat (10 mg/ml in Dimethylformamid) versetzt. Die Mischung wird über Nacht bei Raumtemperatur inkubiert. Fluorescierendes PEI wird mittels Gelfiltration an Sephadex G-25® gereinigt. Zur Elution der Säule wird ein Puffer aus 10 mM MES und 150 mM NaCl, pH 6.5 verwendet. Eluiertes PEI kann über sein Streulicht in einer Apparatur zur dynamischen Lichtsreuung nachgewiesen werden, die Fluoresceinmarkierung wird anhand ihrer Absorption detektiert. Fraktionen mit einem konstanten Verhältnis von Fluorescein zu PEI werden vereinigt und für die nachfolgende Beschichtung genutzt.

Herstellung der Liposomen erfolgt wie in Beispiel 1.

Beschichtung mit PSS erfolgt wie in Beispiel 1.

### Beschichtung mit modifiziertem PEI

Fluorescein-markiertes PEI wird mit einer Konzentration von 10 µg/ml in MES-Puffer (10 mM pH 6.5) gelöst. Die mit PSS beschichteten Liposomen werden im gleichen Puffer soweit verdünnt, dass eine Lipidkonzentration von 200 µg/ml erreicht wird. Gleiche Volumina beider Lösungen werden unter Rühren zusammengegeben. Anschließend wird die Suspension mittels Tangentialdialyse aufkonzentriert.

Weitere Beschichtungen können wie in den beiden oberen Schritten aufgebracht werden. Die eingesetzte Menge Polymer bindet hinreichend vollständig an die Partikeloberfläche, so dass keine Reinigungsschritte zwischengeschaltet werden müssen.

### Beispiel 5

### Einschluß eines fluorescierenden Cargos

### Herstellung der Liposomen

400 mg PC aus Soja und 9,7 mg CTAB werden in Ethanol gelöst und unter Vakuum bis zur Trockne eingedampft. Der Lipidfilm wird anschließend mit einer Carboxyfluoresceinlösung (100 mM Carbixyfluorescein, 10 mM MES, 150 mM NaCl pH 6.5) rehydratisiert. Die Suspension wird dann mehrmals durch isopore Polykarbonatmembranen mit einer Porengröße von 0,2 µm gedrückt.

Nicht eingeschlossenes Carboxyfluorescein wird durch Gelfiltration an Sephadex® G25 entfernt.

### Beschichtung

Die Liposomen werden wie in Beispiel 1 mit PSS und PEI beschichtet. Insgesamt werden fünf Schichten aufgebracht, wobei die äußere und die innere Schicht aus PSS bestehen.

### Beispiel 6

### Beschichtung mit PLL in Abhängigkeit von der liposomalen Ladungsdichte

### Herstellung der Liposomen

10-100 mol-% DPPG und ergänzende Mengen DPPC werden in Isopropanol gelöst und unter Vakuum bis zur Trockne eingedampft. Der Lipidfilm wird anschließend in soviel Puffer (10 mM Hepes, 150 mM NaCl pH 7,5) rehydratisiert, dass eine Lipidkonzentration von 25 mM erreicht wird. Die Suspension wird mindestens einmal eingefroren, bei 50°C wieder aufgetaut und dann mehrmals durch isopore Polykarbonatmembranen mit einer Porengröße von 200nm gedrückt.

### Beschichtung mit PLL und Analyse der Strukturen

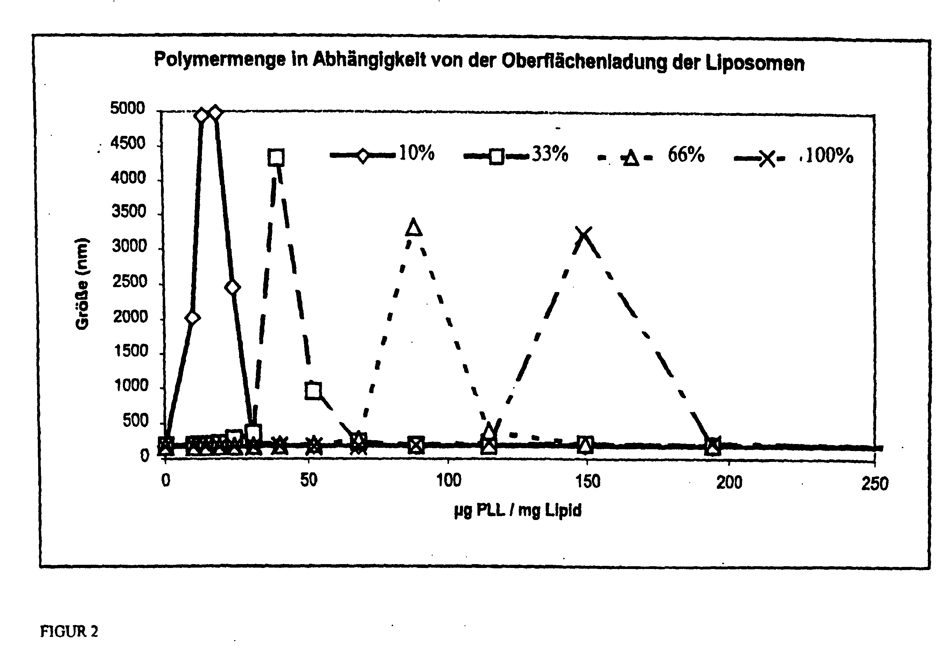

PLL (70- 150 kDa) wird in einer Konzentration von 1 mg/ml in Puffer (10 mM Hepes pH 7,5) gelöst.
Die unterschiedlichen liposomalen Formulierungen werden in Puffer (10 mM Hepes pH 7,5) auf eine Lipidkonzentration von 0,2 mM verdünnt. 0 bis 250 µg PLL/mg Lipid werden in höchstens 0,2 ml Volumen vorgelegt und unter Rütteln mit 1ml der liposomalen Formulierungen versetzt. Anschließend werden die entstandenen Strukturen mittels dynamischer Lichtstreuung vermessen (siehe Figur 2).
Die Größe der Liposomen nimmt durch Aggregatbildung mit dem Polymer schnell zu und überschreitet dann ein Maximum. Erreicht die Größe mit steigenden Polymermengen wieder den ursprünglichen Wert, dann wird die optimal zur Beschichtung geeignete Menge verwendet.

### Beispiel 7

### Beschichtung mit PLL in Abhängigkeit von der Salzkonzentration

### Herstellung der Liposomen

Wie in Beispiel 6, ergänzend werden auch Liposomen mit 0...40% CHEMS und ergänzenden Mengen DPPC hergestellt.

### Beschichtung mit PLL und Analyse der entstandenen Strukturen

PLL wird in geeigneten Konzentrationen (0-230 µg/ml) in Puffer (10 mM Hepes pH 7,5) gelöst. Die liposomalen Formulierungen werden in Puffer (10 mM Hepes pH 7,5) auf eine Lipidkonzentration von 0,2 mM verdünnt. Natriumchlorid-Lösungen von 0 bis 5 M werden in Puffer (10 mM Hepes, pH 7,5) hergestellt. In 96-well-Mikrotiterplatten wird eine Polymer-Salz-Matrix mit je 30 µl der verschiedenen PLL- bzw. NaCl-Lösungen aufgebaut. Die Kavitäten einer Platte werden mit jeweils 240 µl einer liposomalen Formulierung versetzt und die Trübung bei 405 nm nach 10 Minuten gemessen.

Die folgende Tabelle bezeichnet die geeignete Polymermenge (µg PLL/mg Lipid), die zur Generierung stabiler, nicht aggregierter Strukturen benötigt wird.

| Liposomaler | | | | | | | |
|---|---|---|---|---|---|---|---|
| Ladungsträge Salzkonzentration | | | | | | | |
| r | | | | | | | |
| | 10mM | 25mM | 50mM | 75mM | 100mM | 150mM | 300mM |
| 10 % DPPG | 25 | 100 | Aggr. | Aggr. | Aggr. | Aggr. | Aggr. |
| 33 % DPPG | 60 | 100 | 130 | 130 | 150 | 250 | Aggr. |
| 66 % DPPG | 130 | 150 | 150 | 150 | 150 | 170 | Aggr. |
| 100 % DPPG | 220 | 230 | 230 | 230 | 250 | 270 | >300 |
| 10 % CHEMS | 25 | Aggr. | Aggr. | Aggr. | Aggr. | Aggr. | Aggr. |
| 20 % CHEMS | 25 | 70 | Aggr. | Aggr. | Aggr. | Aggr. | Aggr. |
| 30 % CHEMS | 60 | 70 | 100 | 250 | Aggr. | Aggr. | Aggr. |
| 40 % CHEMS | 70 | 110 | 115 | 140 | 240 | Aggr. | Aggr. |

### Salzstabilität der PLL-beschichteten Liposomen

Liposomen, welche bei einer bestimmten Salzkonzentration mit einer geeigneten PLL-Menge beschichtet werden, so dass stabile Strukturen erhalten werden, sind bei Erhöhung der Salzkonzentration instabil und bilden Aggregate.

### Beispiel 8

### Nanokapseln aus Albumin und Heparin

### Herstellung der Liposomen

20 mol-% DPPC und 80 mol-% DPPG werden in Isopropanol gelöst und unter Vakuum bis zur Trockene eingedampft. Der Lipidfilm wird anschließend in soviel Puffer (10 mM Hepes, 150 mM NaCl pH 7,5) rehydratisiert, dass eine Lipidkonzentration von 25 mM erreicht wird. Die Suspension wird mindestens einmal eingefroren, bei 50°C wieder aufgetaut und dann mehrmals durch isopore Polykarbonatmembranen mit einer Porengröße von 200 nm gedrückt.

### Beschichtung mit Albumin und Heparin und Analyse der Strukturen

Die Polymere werden in den Konzentrationen von 1 mg/ml und 5 mg/ml in Puffer (10 mM Natriumacetat, pH 4) gelöst. Die Liposomen werden in Puffer (10 mM Natriumacetat, pH 4) auf eine Lipidkonzentration von 0,2 mM verdünnt. Zu 50 ml dieser verdünnten Liposomen werden nacheinander geeignete Mengen der beiden Polymere (siehe Tabelle) zugemischt. Die jeweils eingesetzte Menge Polymer bindet hinreichend vollständig an die Partikeloberfläche, so dass keine Reinigungsschritte zwischengeschaltet werden müssen.

| Schicht (S) | mg Polymer/mg Lipid |
|---|---|
| S1 BSA | 1,00 |
| S2 Heparin | 0,33 |
| S3 BSA | 4,75 |
| S4 Heparin | 1,59 |
| S5 BSA | 12,66 |
| S6 Heparin | 4,22 |

### Vernetzung der entstandenen Strukturen mit Glutaraldehyd und Aufkonzentrierung

Die erhaltenen Strukturen werden zur Vernetzung mit Glutaraldehyd versetzt. Die Reaktion erfolgt über 2h bei 37°C und einer Endkonzentration von 0,15% Glutaraldehyd. Dann wird mittels 1 M NaOH die Lösung auf pH 7,5 eingestellt. Anschließend wird die Suspension mittels Tangentialdialyse gegen 100mM NaCl dialysiert und dann konzentriert.

### Salzstabilität der vernetzten Strukturen

Die Stabilität der erhaltenen Strukturen wird durch Zugabe von NaCl geprüft. Die Partikel sind mindestens gegenüber 150 mM NaCl beständig.

### Beispiel 9

### Nanokapseln aus PLL und PAS

### Herstellung der Liposomen

60 mol-% DOPE und 40 mol-% CHEMS werden in Isopropanol/Chloroform (3/1) gelöst und unter Vakuum bis zur Trockene eingedampft. Der Lipidfilm wird anschließend in soviel Puffer (10 mM Hepes, 150 mM NaCl pH 7,5) rehydratisiert, dass eine Lipidkonzentration von 25 mM erreicht wird. Die Suspension wird mindestens einmal eingefroren, bei RT wieder aufgetaut und dann mehrmals durch isopore Polykarbonatmembranen mit einer Porengröße von 200 nm gedrückt.

### Beschichtung mit PLL und PAS und Analyse der Strukturen

PLL (Mr 70...150 kDa) und PAS (Mr 30 kDa) werden in den Konzentrationen 1mg/ml und 5mg/ml in Puffer (10 mM Hepes, 100 mM NaCl, pH 7,5) gelöst. Die Liposomen werden in Puffer (10 mM Hepes, 100 mM NaCl, pH 7,5) auf eine Lipidkonzentration von 0,2mM verdünnt. Für die Herstellung der ersten Schicht werden 130 µg PLL/mg Lipid in höchstens 1ml Volumen vorgelegt und 50ml der Liposomen zugespritzt. Für die Herstellung der zweiten Schicht werden 55 µg PAS/mg Lipid vorgelegt und die mit PLL beschichteten Liposomen zugespritzt. Mit den folgenden Schichten wird analog verfahren (siehe Tabelle). Die jeweils eingesetzte Menge Polymer bindet hinreichend vollständig an die Partikeloberfläche, so dass keine Reinigungsschritte zwischengeschaltet werden müssen.

| Schicht (S) | µg Lipid | Polymer/mg |
|---|---|---|
| S1 PLL | 130 | |
| S2 PAS | 55 | |
| S3 PLL | 200 | |
| S4 PAS | 200 | |
| S5 PLL | 850 | |
| S6 PAS | 800 | |

### Vernetzung der entstandenen Strukturen mit EDC und Aufkonzentrierung

Die entstandenen Strukturen werden zur Vernetzung mit EDC versetzt. Die Reaktion erfolgt über 12h bei RT und einer Endkonzentration von 50 mM EDC. Dann wird die Vernetzungsreaktion mit Kaliumacetat (Endkonzentration 100 mM) gestoppt. Anschließend wird die Suspension mittels Tangentialdialyse dialysiert und dann konzentriert.

### Salzstabilität der entstandenen Strukturen

Die Stabilität der erhaltenen Strukturen wird durch Zugabe von NaCl geprüft. Die Partikel sind mindestens gegenüber 150 mM NaCl beständig.

### Beispiel 10

### Verträglichkeit der Strukturen bei pharmazeutischen Anwendungen

Liposomen mit chemisch vernetzten Polyelktrolythüllen werden wie in Beispiel 8 oder 9 hergestellt.

Wistar-Ratten (männlich, 250...300g) werden mit regelmäßigem Tag-Nacht-Rhythmus und bei Futter ad libitum gehalten. Je zwei Tiere werden narkotisiert und erhalten 500 µl der Partikelsuspensionen über die Schwanzvene. Die Tiere werden über verschieden lange Zeiten beobachtet und anschließend dekapitiert und seziert. Zur Injektion wurden im einzelnen verwendet: Beispiel 8, S4 und S5 sowie Beispiel 9, S6.

Alle behandelten Tiere überlebten die Injektion für mindestens 24 Stunden. Bei keinem der Tiere wurde ein abweichendes. Verhalten vom Normalfall festgestellt. Ebenfalls konnten keine krankhaften Veränderungen der Organe festgestellt werden.

### Beispiel 11

### Beschichtung einer Emulsion

2g Olivenöl, 8,5g Wasser, 120mg Phosphatidylcholin und 250mg Glycerol werden gemischt und für zwei Stunden gerührt. Anschließend wird die Emulsion im Ultraschallbad homogenisiert und einmal durch einen Polycarbonatfilter mit einer Porenweite von 200nm extrudiert. Es entsteht eine Emulsion mit einer durchschnittlichen Partikelgröße von 315 nm.

PLL (70-150 kDa) wird in einer Konzentration von 1mg/ml in Puffer (10mM Hepes pH 7,5) gelöst.

40 µl der Emulsion werden in 10ml Puffer (10mM Hepes pH 7,5) verdünnt. 0 bis 50 µg PLL werden vorgelegt und unter Rütteln mit 1ml der Emulsion versetzt. Anschließend werden die entstandenen Strukturen mittels dynamischer Lichtstreuung vermessen. Die Größe der Partikel nimmt durch Aggregatbildung mit dem Polymer schnell zu und überschreitet dann ein Maximum. Erreicht die Größe mit steigenden Polymermengen wieder den ursprünglichen Wert, dann wird die optimal zur Beschichtung geeignete Menge verwendet. Geeignete Menge weiterer Polyelektrolyte für den folgenden Schichtaufbau werden ebenfalls mit dieser Methode bestimmt.

Nachfolgend soll die erfindungsgemäße Vorrichtung anhand der Zeichnung in einem Ausführungsbeispiel näher erläutert werden. In der Zeichnung ist der prinzipielle Aufbau der Vorrichtung in einem Blockschaltbild dargestellt.

Die Hauptflussstrecke der zu beschichtenden Nano- oder Templatpartikel wird von einer den Hauptfluss bewirkenden Pumpe 10, einem dieser Pumpe nachgeschalteten Dämpfungsglied 20 und den Mischern 30, 31, 32, 33, 34, 3X gebildet, wobei zwischen diesen Mischern jeweils ein Zeitglied 41, 42, 43, 44, 45, 4X angeordnet ist.

Die Zuflussstrecken für das Zuführen der einzelnen Polyelektrolyten A, B, C, D, E, X werden jeweils von einer Pumpe 11, 12, 13, 14, 15, 1X sowie jeweils einem zwischen der Pumpe und den Mischern 30 bzw. 31 bzw. 32 bzw. 33 bzw. 34 bzw. 3X angeordneten Dämpfungsglied 21 bzw. 22 bzw. 23 bzw. 24 bzw. 25 bzw. 2X gebildet.

Die einzelnen Baugruppen (Pumpen, Dämpfungsglieder, Mischer und Zeitglieder) sind in ihrer Anordnung mittels Schlauchleitungen verbunden. Das Volumen der jeweiligen Schlauchleitung zwischen dem Mischer 30 und 31 bzw. 31 und 32 bzw. 32 und 33 bzw. 33 und 34 bzw. 34 und 3X bildet das Zeitglied 41 bzw. 42 bzw. 43 bzw. 44 bzw. 45 bzw. 4X. Die Vorrichtung ist durch das Aneinanderreihen weiterer Zuflussstrecken (in der Zeichnung mit unterbrochenen Linien dargestellt) beliebig erweiterbar.

Für die Beschichtung von Liposomen mit mehreren Polyelektrolythüllen wird die Liposomenlösung mit einer Pumpe in einem konstanten Fluss durch die Vorrichtung geführt. Die zur Beschichtung benutzten Polyelektrolyte werden nacheinander in das System der Hauptflussstrecke eingeführt. Die Mischung der Reaktanden erfolgt mittels der Mischer 30, 31, 32, 33, 34, 3X an den Zuführungspunkten. Das Volumen der Schlauchleitungen zwischen den einzelnen Mischern 30 bis 3X ist dabei so bemessen, dass bei der gegebenen Fließgeschwindigkeit eine hinreichend lange Reaktionszeit bis zur Erreichung des nächsten Mischers zur Verfügung steht.

## Patentansprüche

1. Verfahren zur kontinuierlichen Herstellung von Nano- oder Mikrokapseln mit einem Durchmesser von 20 nm bis 40 µm,
**dadurch gekennzeichnet, dass**,
- Templatpartikel in einem wässrigen Medium vorgelegt,
- mit einem Polyelektrolyten elektrisch umgeladen werden,
- ohne Trenn- oder Waschschritte mit einem komplementär zum ersten Polyelektrolyten geladenen zweiten. Polyelektrolyten wieder umgeladen werden,
- und dieser Prozeß mit alternierend geladenen Polyelektrolyten gegebenenfalls weiter fortgesetzt wird.

2. Verfahren nach Anspruch 1,
**dadurch gekennzeichnet, dass**
ein Reaktionszyklus in weniger als 20 Minuten, bevorzugt in weniger als 5 Minuten, besonders bevorzugt in weniger als einer Minute durchlaufen wird.

3. Verfahren nach einem der Ansprüche 1 oder 2,
**dadurch gekennzeichnet, dass**
bei einer Beschichtungsreaktion oder nach deren Abschluß ein chemischer Vernetzer zugesetzt wird.

4. Verfahren nach einem der vorhergehenden Ansprüche,
**dadurch gekennzeichnet, dass**
die Templatpartikel eine Größe zwischen 20 nm und 1000 nm, bevorzugt zwischen 50 nm und 500 nm und besonders bevorzugt zwischen 70 nm und 300 nm besitzen.

5. Verfahren nach einem der vorhergehenden Ansprüche,
**dadurch gekennzeichnet, dass**
in einer Schicht zwei oder mehr voneinander verschiedene Polyelektrolyte gleichzeitig oder nacheinander aufgebracht werden.

6. Verfahren nach einem der vorhergehenden Ansprüche,
**dadurch gekennzeichnet, dass**
eine Salzkonzentration von mehr als 50 mM in einer wässrigen Lösung verwendet wird.

7. Verfahren nach einem der vorhergehenden Ansprüche,
**dadurch gekennzeichnet, dass**
die Templatpartikel Liposomen sind.

8. Verfahren nach einem der vorhergehenden Ansprüche,
**dadurch gekennzeichnet, dass**
die Liposomen nach der Beschichtung aufgelöst werden, vorzugsweise mit einem Detergens.

9. Verfahren nach einem der vorhergehenden Ansprüche,
**dadurch gekennzeichnet, dass**
in die Liposomen Wirkstoffe eingeschlossen sind.

10. Verfahren nach einem der vorhergehenden Ansprüche,
**dadurch gekennzeichnet, dass**
zur Herstellung der Liposomen Phosphatidylserin, Phosphatidylglycerol, Phosphatidylinositol, Phosphatidsäure, Sphingolipide, Ceramide, Tetraetherlipide, Cholesterolsulfat, Cholesterolhemisuccinat, Dimethylaminoethylcarbamoyl-Cholesterol, Alkylcarbonsäure, Alkylsulfonsäuren, Alkylamine, Alkylammoniumsalze, Dialkylamine, N-[1-(2,3 Dioleoy(oxy)propyl]-N,N,N-trimethylammoniumchlorid, Phosphorsäureester mit langkettigen Alkoholen, Phosphatidylcholin, Phosphatidylethanolamin und/oder α-Tocopherol eingesetzt werden.

11. Verfahren nach einem der vorhergehenden Ansprüche,
**dadurch gekennzeichnet, dass**
die Beschichtung mit den Polymeren bei einer Lipidkonzentration kleiner 2 mM bevorzugt kleiner 1mM, besonders bevorzugt kleiner 0,5 mM und ganz besonders bevorzugt kleiner 0,2. mM durchgeführt wird.

12. Verfahren nach einem der vorhergehenden Ansprüche,
**dadurch gekennzeichnet, dass**
Liposomen mit 10 bis 50 Mol%, bevorzugt 30 bis 50 Mol% und besonders bevorzugt 35 und 45 Mol% geladenen Sterolen verwendet werden.

13. Verfahren nach einem der vorhergehenden Ansprüche,
**dadurch gekennzeichnet, dass**
mehr als 10 Mol%, bevorzugt mehr als 40 Mol% und besonders bevorzugt mehr als 60 Mol% Phospholipide verwendet werden.

14. Verfahren nach einem der vorhergehenden Ansprüche,
**dadurch gekennzeichnet, dass**
die Lipidmembran oberhalb ihrer jeweiligen Phasenübergangstemperatur vorliegt.

15. Verfahren nach einem der vorhergehenden Ansprüche,
**dadurch gekennzeichnet, dass**
die Templatpartikel als eine Öl-in-Wasser-Emulsion vorliegen.

16. Verfahren nach einem der vorhergehenden Ansprüche,
**dadurch gekennzeichnet, dass**
die Emulsionen in ihrer Ölphase Wirkstoffe enthalten.

17. Strukturen in Form von Nanokapseln, hergestellt nach einem oder mehreren der Ansprüche 1 bis 16, mit mehr als zwei Polyelektrolytschichten, wobei die Templatpartikel Liposomen oder Emulsionen sind.

18. Strukturen nach Anspruch 17,
**dadurch gekennzeichnet, dass**
im Inneren der Struktur eine oder mehrere Lipidschichten sind.

19. Strukturen nach Anspruch 18,
**dadurch gekennzeichnet, dass**
im Inneren der Struktur eine nicht Wasser-mischbare Ölphase ist.

20. Strukturen nach einem der vorhergehenden Ansprüche,
**dadurch gekennzeichnet, dass**
im Inneren der Struktur Wirkstoffe sind.

21. Strukturen nach einem der vorhergehenden Ansprüche,
**dadurch gekennzeichnet, dass**
die Wirkstoffe Bestandteil einer Hüllschicht sind.

22. Strukturen nach einem der vorhergehenden Ansprüche,
**dadurch gekennzeichnet, dass**
die Wirkstoffe Katalysatoren, Biokatalysatoren, Pharmaka, Proteine, Oligonucleotide, Nucleinsäuren, Kristalle und/oder Sensormoleküle sind.

23. Verwerdung der Strukturen nach einem der Ansprüche 17 bis 22 als Container oder Transporteur bei pharmazeutischen Zubereitungen.

24. Verwendung der Strukturen nach einem der Ansprüche 17 bis 22 zur biochemischen Diagnostik.

25. Verwendung der Strukturen nach einem der Ansprüche 17 bis 22 zur Herstellung von Mikrokristallen, Herbiziden, Pestiziden und/oder Pigmenten.

26. Vorrichtung zur kontinuierlichen Beschichtung von Liposomen mit mehreren Polyelektrolytschichten,
**dadurch gekennzeichnet, dass**
in dem von einer Pumpe (10) bewirkten Hauptfluss der Liposomen nach einem Dämpfungsglied (20) für jeden getrennt zugeführten, von einer Pumpe (11 bis 1X) bewirkten Zufluss des aufzubringenden Polyelektrolyts (A-X), ein Mischer (30-3X) vorgesehen ist, wobei zwischen den Mischern (30-3X) jeweils ein Zeitglied (41-4X) angeordnet ist und zwischen den Pumpen (11-1X) für das Einbringen der Polyelektrolyte (A-X) und den Mischern (30-3X) jeweils ein Dämpfungsglied (21-2X) angeordnet ist.

27. Vorrichtung nach Anspruch 26,
**dadurch gekennzeichnet, dass**
die in der Hauptflussstrecke angeordneten Mischer (3O-3X) mittels einer Schlauchleitung verbunden sind und das Volumen der jeweiligen Schlauchleitung zwischen dem Mischer(30 und 31; bzw. 31 und 32; bzw. 32 und 33; bzw. 33 und 34; bzw. 34 und 3X) das Zeitglied (41 bzw. 42 bzw. 43 bzw. 44 bzw. 45 bzw. 4X) bildet.

## Claims

1. A method for the continuous production of nano- or microcapsules having a diameter of from 20 nm to 40 µm, **characterized in that**
- template particles are provided in an aqueous medium,
- are electrically recharged with a polyelectrolyte,
- are re-recharged without separation or washing steps using a second polyelectrolyte having a complementary charge with respect to the first polyelectrolyte,
- and said process is optionally continued with alternately charged polyelectrolytes.

2. The method according to claim 1, **characterized in that** one reaction cycle is completed within less than 20 minutes, preferably within less than 5 minutes, and more preferably within less than one minute.

3. The method according to claim 1 or 2, **characterized in that** a chemical crosslinker is added during a coating reaction or subsequent to completion thereof.

4. The method according to any of the preceding claims, **characterized in that** the template particles have a size of between 20 nm and 1000 nm, preferably between 50 nm and 500 nm, and more preferably between 70 nm and 300 nm.

5. The method according to any of the preceding claims, **characterized in that** two or more dissimilar polyelectrolytes are applied simultaneously or successively in one layer.

6. The method according to any of the preceding claims, **characterized in that** a salt concentration of more than 50 mM in an aqueous solution is used.

7. The method according to any of the preceding claims, **characterized in that** the template particles are liposomes.

8. The method according to any of the preceding claims, **characterized in that** the liposomes are dissolved subsequent to coating, preferably using a detergent.

9. The method according to any of the preceding claims, **characterized in that** the liposomes have active substances entrapped therein.

10. The method according to any of the preceding claims, **characterized in that** phosphatidyl serine, phosphatidyl glycerol, phosphatidyl inositol, phosphatidic acid, sphingolipids, ceramides, tetraether lipids, cholesterol sulfate, cholesterol hemisuccinate, dimethylamino ethylcarbamoyl cholesterol, alkylcarboxylic acid, alkylsulfonic acids, alkylamines, alkylammonium salts, dialkylamines, N-[1-(2,3-dioleoyl(oxy)propyl]-N,N,N-trimethylammonium chloride, phosphoric acid esters with long-chain alcohols, phosphatidyl choline, phosphatidyl ethanolamine, and/or α-tocopherol are used in the production of the liposomes.

11. The method according to any of the preceding claims, **characterized in that** coating with polymers is performed at a lipid concentration of lower than 2 mM, preferably lower than 1 mM, more preferably lower than 0.5 mM, and most preferably lower than 0.2 mM.

12. The method according to any of the preceding claims, **characterized in that** liposomes including 10 to 50 mole-%, preferably 30 to 50 mole-%, more preferably 35 to 45 mole-% of charged sterols are used.

13. The method according to any of the preceding claims, **characterized in that** more than 10 mole-%, preferably more than 40 mole-%, and more preferably more than 60 mole-% of phospholipids are used.

14. The method according to any of the preceding claims, **characterized in that** the lipid membrane is present above its respective phase transition temperature.

15. The method according to any of the preceding claims, **characterized in that** the template particles are present in the form of an oil-in-water emulsion.

16. The method according to any of the preceding claims, **characterized in that** the emulsions include active substances in their oil phase.

17. Structures in the form of nanocapsules, produced according to one or more of claims 1 to 16, having more than two polyelectrolyte layers wherein the template particles are liposomes or emulsions.

18. The structures according to claim 17, **characterized in that** one or more lipid layers are situated in the interior of said structure.

19. The structures according to claim 18, **characterized in that** a water-immiscible oil phase is situated in the interior of said structure.

20. The structures according to any of the preceding claims, **characterized in that** active substances are situated in the interior of said structure.

21. The structures according to any of the preceding claims, **characterized in that** the active substances are part of a coat layer.

22. The structures according to any of the preceding claims, **characterized in that** the active substances are catalysts, biocatalysts, pharmaceutical agents, proteins, oligonucleotides, nucleic acids, crystals, and/or sensor molecules.

23. Use of the structures according to any of claims 17 to 22 as containers or vehicles in pharmaceutical formulations.

24. Use of the structures according to any of claims 17 to 22 in biochemical diagnostics.

25. Use of the structures according to any of claims 17 to 22 in the production of microcrystals, herbicides, pesticides, and/or pigments.

26. A device for the continuous coating of liposomes with multiple polyelectrolyte layers, **characterized in that** a mixer (30-3X) is provided in the main flow of the liposomes effected by a pump (10), downstream of an attenuator (20) for each separately supplied inflow of required polyelectrolyte (A-X) effected by a pump (11-1X), one timing element (41-4X) at a time being arranged between the mixers (30-3X), and one attenuator (21-2X) at a time being arranged between the pumps (11-1X) for feeding polyelectrolyte (A-X) and the mixers (30-3X).

27. The device according to claim 26, **characterized in that** the mixers (30-3X) arranged in the main flow section are connected by means of a tube line, and that the volume of the respective tube line between the mixer (30 and 31, 31 and 32, 32 and 33, 33 and 34, 34 and 3X, respectively) forms the timing element (41, 42, 43, 44, 45, and 4X, respectively).

## Revendications

1. Procédé de préparation en continu de microcapsules ou de nanocapsules d'un diamètre de 20 nm à 40 µm,
**caractérisé en ce**
- **qu'**on introduit les particules de matrice dans un milieu aqueux,
- on inverse la charge électrique avec une polyélectrolyte,
- on inverse à nouveau la charge électrique avec une seconde polyélectrolyte ayant une charge opposée à la première polyélectrolyte, sans utilisant des étapes de séparation ou de lavage,
- et on poursuit éventuellement ce processus en utilisant des polyélectrolytes de charge alternante.

2. Procédé selon la revendication 1,
**caractérisé en ce**
**qu'**on effectue un cycle de réaction en moins de 20 minutes, de préférence en moins de 5 minutes, et de manière encore plus préférée en moins d'une minute.

3. Procédé selon une des revendications 1 ou 2,
**caractérisé en ce**
**qu'**on ajoute un agent chimique de réticulation au cours d'une réaction d'enrobage ou après la fin de cette réaction.

4. Procédé selon une des revendications précédentes,
**caractérisé en ce que**
les particules de matrice ont une taille comprise entre 20 nm et 1000 nm, de préférence entre 50 nm et 500 nm, et de manière encore plus préférée entre 70 nm et 300 nm.

5. Procédé selon une des revendications précédentes,
**caractérisé en ce**
**qu'**on applique dans une couche deux ou plusieurs polyélectrolytes différentes l'une de l'autre, de manière simultanée ou l'une après l'autre.

6. Procédé selon une des revendications précédentes,
**caractérisé en ce**
**qu'**on utilise une concentration de sel supérieure à 50 mM dans une solution aqueuse.

7. Procédé selon une des revendications précédentes,
**caractérisé en ce que**
les particules de matrice sont des liposomes.

8. Procédé selon une des revendications précédentes,
**caractérisé en ce**
**qu'**on dissous les liposomes après l'enrobage, de préférence avec un détergent.

9. Procédé selon l'une des revendications précédentes,
**caractérisé en ce que**
des agents actifs sont insérés dans les liposomes.

10. Procédé selon l'une des revendications précédentes,
**caractérisé en ce que**,
pour préparer les liposomes, on utilise de la phosphatidylsérine, du phosphatidylglycérol, du phosphatidylinositol, de l'acide phosphatidique, des sphingolipides, des céramides, des tétraétherlipides, du cholestérol sulfate, du cholestérol hémisuccinate, du diméthylaminoéthylcarbamoylcholestérol, des acides alkylcarboxyliques, des acides alkylsulfoniques, des alkylamines, des sels d'alkylammonium, des dialkylamines, du N-[1-(2,3-dioléoyl(oxy)propyl]-N,N,N-triméthylammonium chlorure, des esters d'acide phosphorique avec des alcools à longue chaîne, de la phosphatidylcholine, du phosphatidyléthanolamine et/ou du α-tocophérol.

11. Procédé selon l'une des revendications précédentes,
**caractérisé en ce**
**qu'**on réalise l'enrobage avec les polymères à une concentration de lipide inférieure à 2 mM, de préférence inférieure à 1 mM, de manière plus préférée inférieure à 0,5 mM et de manière encore plus préférée inférieure à 0,2 mM.

12. Procédé selon l'une des revendications précédentes,
**caractérisé en ce**
**qu'**on utilise des liposomes contenant 10 à 50 % en moles, de préférence 30 à 50 % en moles et de manière encore plus préférée de 35 à 45 % en moles de stérols chargés.

13. Procédé selon l'une des revendications précédentes,
**caractérisé en ce**
**qu'**on utilise des phospholipides à une teneur supérieure à 10 % en moles, de préférence à une teneur supérieure à 40 % en moles et de manière encore plus préférée à une teneur supérieure à 60 % en moles.

14. Procédé selon l'une des revendications précédentes,
**caractérisé en ce que**
la membrane du lipide se forme à une température supérieure à sa température respective de transition des phases.

15. Procédé selon l'une des revendications précédentes,
**caractérisé en ce que**
les particules de matrice sont sous forme d'une émulsion huile-dans-eau.

16. Procédé selon l'une des revendications précédentes,
**caractérisé en ce que**
les émulsions contiennent des agents actifs dans leur phase huile.

17. Structures en forme de nanocapsules, préparées selon une ou plusieurs des revendications 1 à 16, moyennant quoi les particules de matrice sont des liposomes ou des émulsions.

18. Structures selon la revendication 17,
**caractérisées en ce**
**qu'**il y a une ou plusieurs couches de lipide à l'intérieur de la structure.

19. Structures selon la revendication 18,
**caractérisées en ce**
**qu'**une phase huile non miscible à l'eau se trouve à l'intérieur de la structure.

20. Structures selon l'une des revendications précédentes,
**caractérisées en ce que**
des agents actifs se trouvent à l'intérieur de la structure.

21. Structures selon l'une des revendications précédentes,
**caractérisées en ce que**
les agents actifs sont un constituant d'une couche d'enveloppe.

22. Structures selon l'une des revendications précédentes,
**caractérisées en ce que**
les agents actifs sont des catalyseurs, des biocatalyseurs, des agents pharmaceutiques, des protéines, des oligonucléotides, des acides nucléiques, des cristaux et/ou des molécules capteurs.

23. Utilisation des structures selon une des revendications 17 à 22 en tant que conteneur ou transporteur pour des préparations pharmaceutiques.

24. Utilisation des structures selon une des revendications 17 à 22 pour réaliser un diagnostic biochimique.

25. Utilisation des structures selon une des revendications 17 à 22 pour préparer des microcristaux, des herbicides, des pesticides et/ou des pigments.

26. Dispositif d'enrobage en continu de liposomes avec plusieurs couches de polyélectrolyte,
**caractérisé en ce que**
dans le flux principal des liposomes alimenté par une pompe (10), après un atténuateur (20), pour chaque admission de la polyélectrolyte à appliquer (A-X) alimentée séparément par une pompe (11 à 1X), un mélangeur (30-3X) est prévu, moyennant quoi, entre les mélangeurs (30-3X), un organe temporisateur (41-4X) est disposé à chaque fois et entre les pompes (11-1X) d'admission de polyélectrolyte (A-X) et les mélangeurs (30-3X), à chaque fois un atténuateur (21-2X) est disposé.

27. Dispositif selon la revendication 26,
**caractérisé en ce que**
les mélangeurs (30-3X) disposés dans la trajectoire du flux principal sont reliés par une conduite souple et le volume respectif de la conduite souple entre les mélangeurs (30 et 31, ou 31 et 32, ou 32 et 33, ou 33 et 34, ou 34 et 3X) forme l'organe temporisateur (41 ou 42 ou 43 ou 44 ou 45 ou 4X).
